# EUROPEAN PATENT APPLICATION

(11) **EP 3 278 714 A1**
(43) Date of publication of application: **07.02.2018**
(21) Application number: 16789491.4
(22) Date of filing: 11.04.2016
(51) Int. Cl.: A61B 1/00, G02B 23/24

(54) **ENDOSCOPE SYSTEM**

(30) Priority: 07.05.2015 JP 2015094992
(71) Applicant: Olympus Corporation, Hachioji-shi, Tokyo 192-8507 (JP)
(72) Inventor: WAKE Fuminori, Hachioji-shi Tokyo 192-8507 (JP); HINO Kazuhiko, (JP)
(74) Representative: Schicker, Silvia
(86) International application number: PCT/JP2016/061713
(87) International publication number: WO 2016/178355

(57) **Abstract**

An endoscope system includes an endoscope including an insertion section and an objective lens provided in a distal end portion of the insertion section, a first pipe configured to cause foreign matters that are a cause of obstruction of an observation field of view of the objective lens to flow through, a valve configured to switch the first pipe to either one of an open state and a closed state, a first suction apparatus configured to suck the foreign matters through the valve in a case where the first pipe is in the open state, a second pipe divided from a part of the first pipe not passing through the valve, a cautery apparatus configured to apply energy and perform a surgical treatment on a body tissue in the observation field of view of the objective lens, and a second suction apparatus configured to suck fluid through the second pipe when energy is applied to the body tissue by the cautery apparatus.

## Description

### Technical Field

The present invention relates to an endoscope system, and more particularly, to an endoscope system that is used when performing various treatments on a lesion such as a cancer tissue.

### Background Art

With respect to an endoscope system in a medical field, a method and a configuration are known, which are for removing, when inserting an insertion section of an endoscope into a body cavity and observing a body tissue in the body cavity, fluid in the body cavity, such as mucus in the body cavity and liquid such as cleaning water used to clean an optical system, and solid matters, such as residues in the body cavity and treatment target tissues excised by using a treatment instrument, by using a suction pipe that is provided inside the endoscope and that serves also as a treatment instrument insertion channel to which a suction apparatus is connected, so as to secure a desirable observation field of view of the endoscope.

More specifically, for example, Japanese Patent Application Laid-Open Publication No. 2007-105395 discloses a configuration according to which a suction opening of a suction pipe is opened at an insertion section of an endoscope, a part of the suction pipe is divided and opened as a treatment instrument insertion opening at an operation section of the endoscope, and a suction apparatus is connected, at a connector of the endoscope to an external apparatus, to a pipe sleeve communicating with an opening of the suction pipe, where fluid in a body cavity is sucked through the suction pipe from the suction opening after driving of the suction apparatus.

Now, with respect to an endoscope used in the medical field, a procedure is known, according to which a treatment target tissue is dissected and excised from body tissues in a state where an insertion section is inserted into a body cavity and the treatment target tissue in the body tissues in the body cavity is observed by an optical system provided in the insertion section, by causing a treatment instrument inserted through a suction pipe via a treatment instrument insertion opening provided in an operation section of the endoscope to protrude forward in a longitudinal direction (hereinafter simply referred to as "forward") and applying energy to the treatment target tissue from the protruded treatment instrument.

For example, an endoscopic submucosal dissection (hereinafter referred to as "ESD") is known, which is a procedure of cauterizing, cutting and removing, using a high-frequency knife, which is a cautery apparatus, a cancer tissue which is floated in advance by injection of a dedicated liquid, in a state where an insertion section is inserted into the body cavity and the cancer tissue in the body cavity is observed by an optical system of the insertion section, by causing the high-frequency knife inserted through a suction pipe via a treatment instrument insertion opening to protrude forward from a suction opening on a distal end surface of the insertion section and by causing the insertion section to move forward and backward in the longitudinal direction (hereinafter simply referred to as "forward and backward").

During a procedure of cutting a body tissue and coagulating the blood, such as the ESD, mucus and fat in the body cavity tend to evaporate due to cutting and coagulation performed by application of high-frequency current to a cancer tissue from an electric knife, for example, and the mucus and the fat are turned into mist which is fluid containing solid particle components, or more specifically, gas containing components derived from a body tissue.

As a result, especially with a procedure such as the ESD which is performed over a long period of time, a small body cavity tends to be filled with mist, possibly resulting in a situation where an observation field of view of the endoscope is obstructed. Accordingly, to secure the observation field of view during the ESD, a technique of sucking gas containing components derived from a body tissue from a suction opening by using a suction pipe of an endoscope apparatus disclosed in Japanese Patent Application Laid-Open Publication No. 2007-105395 is desirably used.

However, during the ESD, because a surgeon is performing, for example, an operation of twisting a high-frequency electric knife and an insertion section of an endoscope and an operation of bending a bending portion provided at the insertion section of the endoscope substantially at the same time using both hands, and thus both hands are full, an operation of pressing a suction button for performing a suction operation is difficult to perform. Accordingly, a problem is caused that an excessive burden is imposed on a surgeon performing the ESD.

Note that the problem described above is not limited to the ESD, and the same thing can be said for other methods and configurations for dissecting and excising a treatment target tissue from a body tissue by application of energy to the treatment target tissue from a cautery apparatus.

The present invention has been made in view of the problem described above, and has its object to provide an endoscope system which is capable of smoothly removing fluid filling an inside of a subject and containing solid particle components, and of securing a desirable observation field of view of an endoscope.

### Disclosure of Invention

### Means for Solving the Problem

An endoscope system according to an aspect of the present invention includes an endoscope including an insertion section configured to be inserted into a body cavity of a subject, and an objective lens provided in a distal end portion of the insertion section, a first pipe provided in the insertion section, the first pipe being configured to cause foreign matters that are a cause of obstruction of an observation field of view of the objective lens to flow outside the endoscope, a valve provided at an intermediate portion of the first pipe, the valve being configured to switch the first pipe to either one of an open state and a closed state, a first suction apparatus provided outside the endoscope, the first suction apparatus being configured to perform a suction operation of sucking the foreign matters through the valve in a case where the first pipe is in the open state, a second pipe formed as a pipe divided from a part, of the intermediate portion of the first pipe, not passing through the valve, the second pipe being configured to cause fluid, in the foreign matters, containing a solid particle component to flow outside the endoscope, a cautery apparatus configured to perform a surgical treatment on a body tissue in the observation field of view of the objective lens by applying energy to the body tissue, and a second suction apparatus provided outside the endoscope, the second suction apparatus being configured to perform a suction operation of sucking the fluid through the second pipe when energy is applied to the body tissue by the cautery apparatus.

### Brief Description of the Drawings

Fig. 1 is a diagram showing a configuration of main sections of an endoscope system according to an embodiment;
Fig. 2 is a diagram showing a configuration of main sections of an endoscope system according to a modification of the embodiment;
Fig. 3 is a diagram for describing an example configuration of an air feeding apparatus according to the modification of the embodiment, for a case of performing or stopping an air feeding operation according to a measurement value obtained by measuring an internal pressure of a suction pipe; and
Fig. 4 is a diagram for describing an example configuration of a distal end surface of a distal end portion of an endoscope according to the modification of the embodiment.

### Best Mode for Carrying Out the Invention

Hereinafter, an embodiment of the present invention will be described with reference to the drawings.

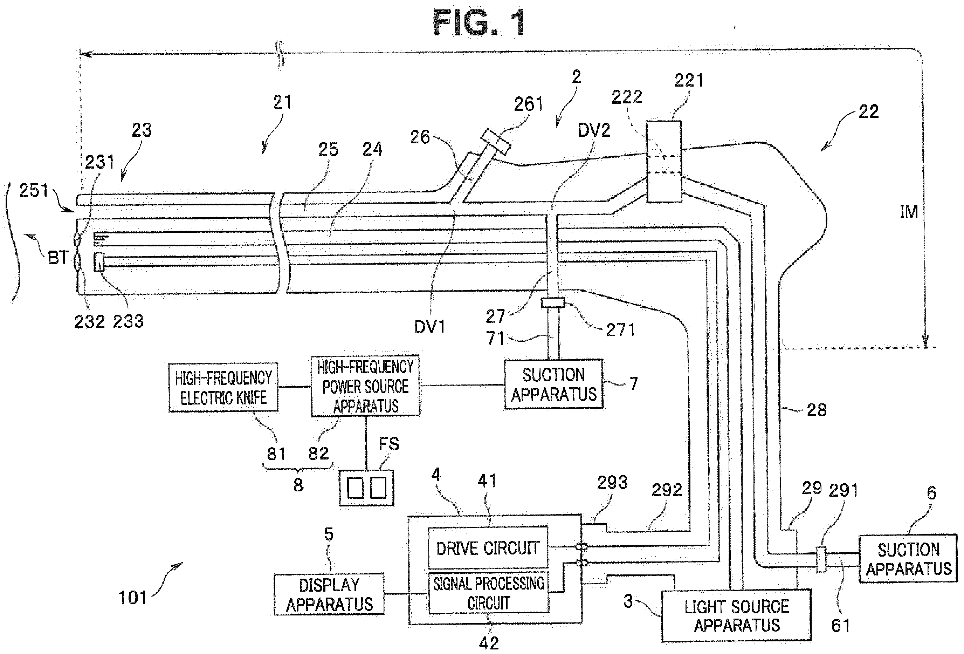

Figs. 1 to 4 relate to an embodiment of the present invention.

As shown in Fig. 1, an endoscope system 101 includes an endoscope 2, which is configured to be insertable into a body cavity of an examinee and to pick up an image of an object such as a body tissue in the body cavity and output an image pickup signal, a light source apparatus 3, which is configured to supply illumination light that is used for observation of the object through a light guide 24 that is inserted and arranged inside the endoscope 2, a processor 4, which is configured to generate and output a video signal according to the image pickup signal outputted from the endoscope 2 and the like, a display apparatus 5, which is configured to display an observation image according to the video signal outputted from the processor 4 and the like, a suction apparatus 6, a suction apparatus 7, and a cautery apparatus 8. Fig. 1 is a diagram showing a configuration of main sections of the endoscope system according to the embodiment.

The endoscope 2 includes an insertion section 21, which is formed into an elongated shape so as to be insertable into a body cavity of an examinee, and an operation section 22, which is provided on a proximal end side of the insertion section 21. And the endoscope 2 is configured to be detachably connected to each of the light source apparatus 3, the processor 4, and the suction apparatus 6 via a universal cable 28 extending from the operation section 22.

More specifically, for example, as shown in Fig. 1, the endoscope 2 is configured to be detachably connected to the light source apparatus 3 via a universal connector 29 at an end portion of the universal cable 28. And for example, as shown in Fig. 1, the endoscope 2 is configured to be detachably connected to the processor 4 via a connector 293 at an end portion of a signal cable 292 extending from the universal connector 29. Moreover, for example, as shown in Fig. 1, the endoscope 2 is configured to be connected to the suction apparatus 6 via a suction pipe sleeve 291 provided on the universal connector 29 and a suction tube 61 detachably connected to the suction pipe sleeve 291.

An illumination lens 231 for irradiating an object with illumination light that is emitted via an emitting end portion of the light guide 24, an objective lens 232 for obtaining an optical image of the object illuminated with the illumination light, and an image pickup device 233 configured to generate an image pickup signal by picking up the optical image of the object obtained by the objective lens 232 are provided in a distal end portion 23 of the insertion section 21.

For example, the image pickup device 233 includes an image sensor such as a CCD or a CMOS. And the image pickup device 233 is electrically connected to the connector 293 via a signal line that is embedded in the endoscope 2. Moreover, when the connector 293 is connected to the processor 4, the image pickup device 233 is driven according to a drive signal that is outputted from the processor 4, and also outputs, to the processor 4, an image pickup signal that is generated by picking up an optical image of the object.

For example, the light guide 24 includes a fiber bundle including a plurality of optical fibers. And when the universal connector 29 is connected to the light source apparatus 3, the light guide 24 transmits illumination light entering from an incident end portion arranged near the light source apparatus 3, and also emits the transmitted illumination light from the emitting end portion arranged near the illumination lens 231.

A forceps channel 25, which is formed as a pipe allowing foreign matters entering an observation field of view of the objective lens 232 during a surgical treatment such as the ESD by the cautery apparatus 8, that is, foreign matters which are a cause of obstruction of the observation field of view of the objective lens 232, to flow outside the endoscope 2, is provided at a part, inside the endoscope 2, from the insertion section 21 to the universal connector 29. Note that the foreign matters include gas containing components derived from a body tissue treated by the cautery apparatus 8, a tissue fragment which is a body tissue excised by a treatment by the cautery apparatus 8, and mucus that is present near a treatment target part by the cautery apparatus 8, for example.

A forceps opening 251 is formed to the forceps channel 25, at the distal end portion 23, the forceps opening 251 having a function as a suction opening for sucking foreign matters which are a cause of obstruction of the observation field of view of the objective lens 232, and a function of a protrusion opening for allowing protrusion of an elongated treatment instrument that is inserted through a treatment instrument insertion pipe 26 described below. And the forceps channel 25 is configured to be connected to the suction apparatus 6 via the suction pipe sleeve 291 and the suction tube 61 when placed in an open state according to operation of a suction switch 221 described below. That is, the forceps channel 25 has a function as a suction pipe that is used for sucking foreign matters which are a cause of obstruction of the observation field of view of the objective lens 232.

The treatment instrument insertion pipe 26 divided from a dividing portion DV1 belonging to an intermediate portion of the insertion section 21 is formed to the forceps channel 25.

The treatment instrument insertion pipe 26 is formed in a manner allowing an elongated treatment instrument inserted from a treatment instrument insertion opening 261 to be inserted to the forceps opening 251 side of the forceps channel 25.

Moreover, a suction pipe 27 divided from a dividing portion DV2 belonging to the intermediate portion of the insertion section 21 and positioned nearer to a proximal end side (operation section 22 side) than the dividing portion DV1 described above is formed to the forceps channel 25.

The suction pipe 27 is configured to be connected to the suction apparatus 7 via a suction pipe sleeve 271 provided on the insertion section 21 of the endoscope 2 and a suction tube 71. And the suction pipe 27 is formed as a pipe allowing gas containing, among foreign matters which are a cause of obstruction of the observation field of view of the objective lens 232, components derived from a body tissue treated by the cautery apparatus 8 to flow outside the endoscope 2.

The operation section 22 has a form allowing a user, such as a surgeon, to grasp and operate. And the operation section 22 is provided with a scope switch, not shown, including at least one switch capable of issuing an instruction to the processor 4 according to an input operation of the user. Moreover, the operation section 22 is provided with a suction switch 221 that is provided at an intermediate portion of the forceps channel 25, and that has a function as a manual valve allowing the forceps channel 25 to be manually switched to one of an open state and a closed state according to a predetermined operation, such as pressing, by the user.

For example, as shown in Fig. 1, a communication tube portion 222, which is formed to have a tubular shape and which is capable of communicating with an intermediate portion IM belonging to a section, of the forceps channel 25, from a mid-portion of the insertion section 21 to a boundary portion of the operation section 22 and the universal cable 28, is embedded in the suction switch 221. And the suction switch 221 is configured to be fixed and arranged at either one of a position where the communication tube portion 222 is interposed at the intermediate portion IM and a position where the communication tube portion 222 is retracted from the intermediate portion IM, with a spring, a stopper or the like, not shown.

Accordingly, for example, when the communication tube portion 222 is interposed at the intermediate portion IM according to operation of the suction switch 221 by the user, the forceps channel 25 is placed in the open state. And for example, when the communication tube portion 222 is retracted from the intermediate portion IM according to operation of the suction switch 221 by the user, the forceps channel 25 is placed in the closed state.

According to the configuration described above, the suction pipe 27 is formed as a pipe that is divided from the dividing portion DV2, which is at a position in the intermediate portion IM of the forceps channel 25 before passing through the communication tube portion 222 of the suction switch 221 when seen from a distal end side of the insertion section 21.

For example, the light source apparatus 3 includes a white light source, and is configured to be able to supply white light emitted from the white light source as illumination light.

The processor 4 includes a drive circuit 41, and a signal processing circuit 42.

For example, the drive circuit 41 is configured to generate a drive signal specifying an exposure period, a read-out period and the like of the image pickup device 233. And the drive circuit 41 is configured to output the generated drive signal as mentioned above to the image pickup device 233 when the connector 293 is connected to the processor 4.

For example, the signal processing circuit 42 is configured to generate a video signal by performing signal processing, such as noise removal, white balance correction and gamma correction, on an image pickup signal that is outputted from the image pickup device 233, and to output the generated video signal to the display apparatus 5, when the connector 293 is connected to the processor 4.

The suction apparatus 6 is provided outside the endoscope 2, and includes a pump for suction and the like. And the suction apparatus 6 is configured to perform a suction operation of sucking a substance from the forceps opening 251 and through the communication tube portion 222 when the suction apparatus 6 is connected to the endoscope 2 via the suction pipe sleeve 291 and the suction tube 61 and the forceps channel 25 is placed in the open state according to operation of the suction switch 221. That is, the suction apparatus 6 is configured to perform, in the case where the forceps channel 25 is in the open state, a suction operation of sucking, through the suction switch 221, foreign matters which are a cause of obstruction of the observation field of view of the objective lens 232.

The suction apparatus 7 is provided outside the endoscope 2, and includes a pump for suction and the like. And the suction apparatus 7 is configured to be able to detect, based on an on/off identification signal that is outputted from a high-frequency power source apparatus 82 as described below, whether a current operation state of the cautery apparatus 8 is an on state or an off state. Moreover, the suction apparatus 7 is configured to perform a suction operation of sucking a substance from the forceps opening 251 and through the dividing portion DV2 and the suction pipe 27 when the suction apparatus 7 is connected to the endoscope 2 via the suction pipe sleeve 271 and the suction tube 71 and energy is applied by the cautery apparatus 8 in a manner described below, that is, when detecting that the current operation state of the cautery apparatus 8 is the on state. Moreover, when the cautery apparatus 8 is not performing an operation of applying energy in a manner described below, that is, when detecting that the current operation state of the cautery apparatus 8 is the off state, the suction apparatus 7 stops the suction operation of sucking a substance from the forceps opening 251 through the dividing portion DV2 and the suction pipe 27.

The cautery apparatus 8 is configured to be able to perform a surgical treatment on a body tissue in the observation field of view of the objective lens 232 by applying energy on the body tissue. More specifically, the cautery apparatus 8 includes a high-frequency electric knife 81, which is an elongated treatment instrument that can be inserted through the treatment instrument insertion pipe 26 and the forceps channel 25, and a high-frequency power source apparatus 82, which is configured to generate high-frequency current that is used for a surgical treatment of a body tissue and to supply the generated high-frequency current to the high-frequency electric knife 81 as electrical energy.

For example, the high-frequency electric knife 81 includes, on a distal end side, an electrode (not shown) for applying the electrical energy supplied from the high-frequency power source apparatus 82. And the high-frequency electric knife 81 includes, on a proximal end side, an operation element (not shown), such as a lever, allowing an operation regarding treatment in a state where the electrode on the distal end side is protruded from the forceps opening 251.

For example, the high-frequency power source apparatus 82 is configured to be switched to either one of an on state in which electrical energy is supplied to the high-frequency electric knife 81 and an off state in which supply of electrical energy to the high-frequency electric knife 81 is stopped, according to a predetermined operation, such as pressing, performed at a foot switch FS. Moreover, the high-frequency power source apparatus 82 is configured to generate an on/off identification signal indicating whether the current operation state of the cautery apparatus 8 is the on state or the off state, and to output the signal to the suction apparatus 7. Moreover, in the on state, the high-frequency power source apparatus 82 is able to supply electrical energy according to one mode selected by operation of the foot switch FS from a plurality of modes including a hemostasis mode in which a body tissue is coagulated and the bleeding is stopped and a cutting mode in which moisture in a body tissue is evaporated and cutting is performed, for example.

That is, the on state described above corresponds to an operation state in which electrical energy according to one mode selected by operation of the foot switch FS is applied to a body tissue by the cautery apparatus 8. And the off state described above corresponds to an operation state in which electrical energy is not applied to a body tissue by the cautery apparatus 8.

Next, a specific operation and the like of the endoscope system 101 according to the present embodiment will be described.

First, after connecting each section of the endoscope system 101 and turning on the power, a user arranges the distal end portion 23 at a position at which a body tissue BT, which is a treatment target body tissue inside a body cavity of an examinee, is in the observation field of view of the objective lens 232, by inserting the insertion section 21 into the body cavity while checking an image that is displayed by the display apparatus 5. That is, in a state where the distal end portion 23 is arranged at such a position, an image allowing the body tissue BT to be seen is displayed by the display apparatus 5.

Then, the user arranges the distal end portion 23 at the position described above, causes the electrode at a distal end portion of the high-frequency electric knife 81 to protrude from the forceps opening 251, and operates the foot switch FS in a state where the electrode is in contact with the body tissue BT, and thereby switches the operation state of the high-frequency power source apparatus 82 from the off state to the on state. Then, according to such operations of the user, electrical energy is applied to the body tissue BT from the electrode at the distal end portion of the high-frequency electric knife 81.

According to operation of the foot switch FS by the user, the high-frequency power source apparatus 82 outputs, to the suction apparatus 7, an on/off identification signal indicating that the current operation state of the cautery apparatus 8 is the on state.

The suction apparatus 7 detects that the current operation state of the cautery apparatus 8 is the on state based on the on/off identification signal outputted from the high-frequency power source apparatus 82, and then, performs a suction operation of sucking a substance from the forceps opening 251 through the dividing portion DV2 and the suction pipe 27. That is, when detecting based on the on/off identification signal outputted from the high-frequency power source apparatus 82 that energy is applied to the body tissue BT by the cautery apparatus 8, the suction apparatus 7 performs a suction operation of sucking, through the suction pipe 27, gas containing components derived from the body tissue treated by the cautery apparatus 8, which is fluid containing solid particle components.

As described above, according to the endoscope system 101 of the present embodiment, the suction apparatus 7 performs the suction operation when electrical energy is supplied from the high-frequency power source apparatus 82 to the high-frequency electric knife 81. Therefore, according to the endoscope system 101 of the present embodiment, gas containing components derived from a body tissue treated by the cautery apparatus 8 may be sucked in without the surgeon performing a special operation. The endoscope system 101 of the present embodiment is thus capable of smoothly removing fluid filling an inside of a subject and containing solid particle components, and of securing a desirable observation field of view of the endoscope.

Note that according to the present embodiment, a porous film or a filter having filtration characteristics that allow only gas to pass through while blocking liquid and solid may be provided at an inlet portion of the suction pipe 27. Such a configuration allows a period of time required to clean an inside of the suction pipe 27 to be reduced.

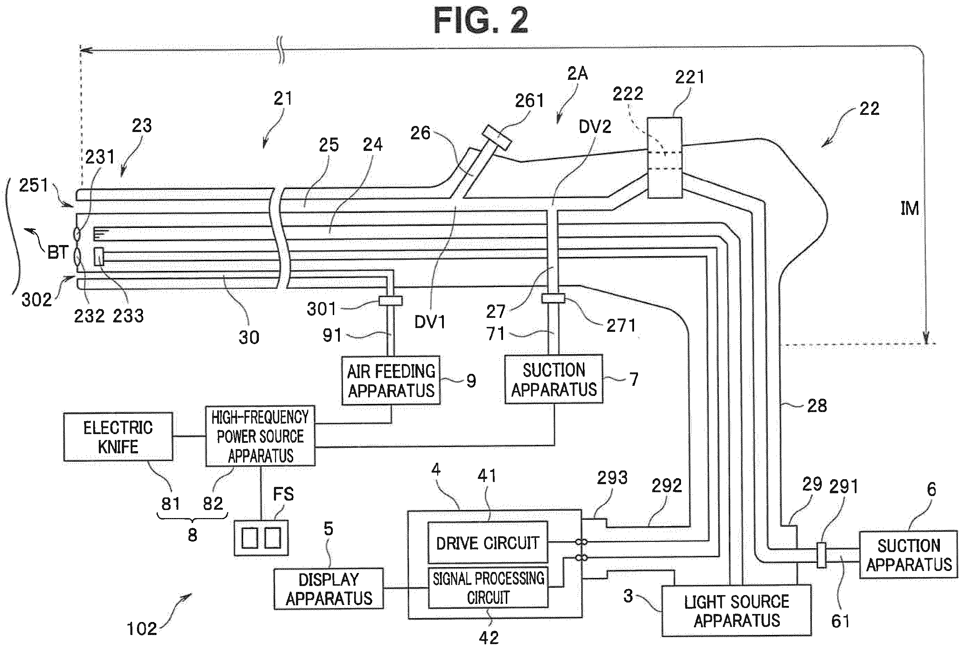

Moreover, the present embodiment is applied to an endoscope system 102 as shown in Fig. 2, for example, in substantially the same manner, without being limited to the endoscope system 101 described above. A configuration and the like of the endoscope system 102 according to such a modification will be described below. Note that in the following, a specific description regarding the configuration and the like described above is omitted as appropriate for the sake of simplicity.

As shown in Fig. 2, the endoscope system 102 includes an endoscope 2A, which is configured to be insertable into a body cavity of an examinee and to pick up an image of an object such as a body tissue in the body cavity and output an image pickup signal, the light source apparatus 3, which is configured to supply illumination light that is used for observation of the object through the light guide 24 that is inserted and arranged inside the endoscope 2A, the processor 4, which is configured to generate and output a video signal according to the image pickup signal outputted from the endoscope 2A and the like, the display apparatus 5, the suction apparatus 6, the suction apparatus 7, the cautery apparatus 8, and an air feeding apparatus 9. Fig. 2 is a diagram showing a configuration of main sections of the endoscope system according to the modification of the embodiment.

The endoscope 2A has a configuration which is substantially the same as the configuration of the endoscope 2 but provided with an air feeding pipe 30, which is formed as a separate pipe different from the forceps channel 25.

The air feeding pipe 30 is provided inside the insertion section 21 of the endoscope 2A, and is formed as a pipe that is capable of causing gas supplied from outside the endoscope 2A to flow through and to be discharged from the distal end portion 23. And the air feeding pipe 30 is configured to be connected to the air feeding apparatus 9 via an air feeding pipe sleeve 301 provided on the insertion section 21 of the endoscope 2A and an air feeding tube 91. And the air feeding pipe 30 is configured to be able to discharge gas supplied from the air feeding apparatus 9 from an air feeding opening 302 provided at the distal end portion 23.

The air feeding apparatus 9 is provided outside the endoscope 2A, and includes a pump for air feeding and the like. And the air feeding apparatus 9 is configured to be able to detect whether the cautery apparatus 8 is in the on state or the off state, based on an on/off identification signal that is outputted from the high-frequency power source apparatus 82. And the air feeding apparatus 9 is configured to perform an air feeding operation of supplying gas to the air feeding pipe 30 when the air feeding apparatus 9 is connected to the endoscope 2A via the air feeding pipe sleeve 301 and the air feeding tube 91 and energy is applied by the cautery apparatus 8 to a body tissue BT in the observation field of view, that is, when detecting that the cautery apparatus 8 is in the on state based on an on/off identification signal that is outputted from the high-frequency power source apparatus 82. Moreover, the air feeding apparatus 9 is configured to stop the air feeding operation for supplying gas to the air feeding pipe 30 when the cautery apparatus 8 is not performing the operation of applying energy to a body tissue BT in the observation field of view, that is, when detecting that the cautery apparatus 8 is in the off state based on an on/off identification signal that is outputted from the high-frequency power source apparatus 82.

As described above, according to the endoscope system 102 of the present modification, the suction operation of the suction apparatus 7 and the air feeding operation of the air feeding apparatus 9 are performed together during supply of electrical energy from the high-frequency power source apparatus 82 to the high-frequency electric knife 81. Therefore, according to the endoscope system 102 of the present modification, in addition to the effect by the endoscope system 101, it is possible to achieve an effect that a pressure inside a body cavity may be kept substantially constant during the ESD while occurrence of excessive negative pressure that is caused by the suction operation of the suction apparatus 7 is suppressed.

Note that in the present modification, the air feeding operation of the air feeding apparatus 9 may be performed or stopped according to a measurement value obtained by measuring an internal pressure of the suction pipe 27, for example, without being limited to a case where the air feeding operation of the air feeding apparatus 9 is performed or stopped based on an on/off identification signal that is outputted from the high-frequency power source apparatus 82.

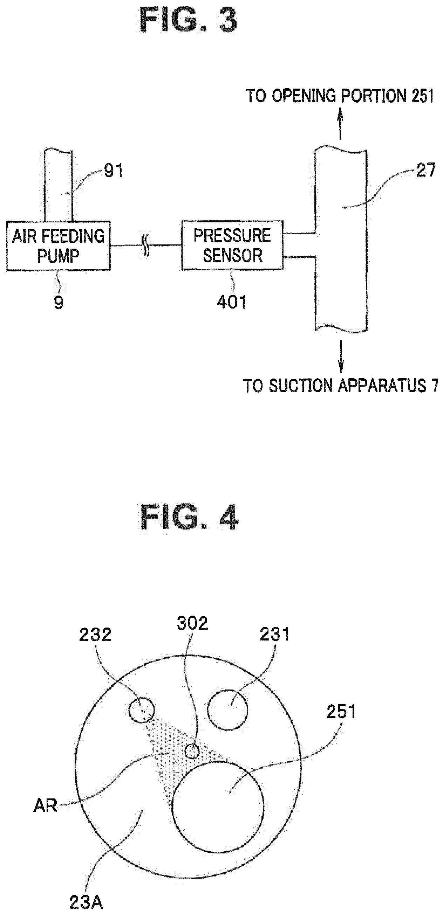

More specifically, for example, the air feeding operation of the air feeding apparatus 9 may be performed or stopped according to an output signal from a pressure sensor 401, which is provided inside the endoscope 2A at a position as shown in Fig. 3 and is configured to measure the internal pressure of the suction pipe 27 and acquire a measurement value, and to generate and output an electrical signal indicating whether the acquired measurement value is a predetermined threshold TH or more, for example. Moreover, in the case where such a pressure sensor 401 is provided inside the endoscope 2A, the air feeding apparatus 9 may perform the air feeding operation when detecting that the measurement value obtained by the pressure sensor 401 is less than the predetermined threshold TH, and may stop the air feeding operation when detecting that the measurement value is the predetermined threshold TH or more, for example. According to such a configuration, the high-frequency power source apparatus 82 and the air feeding apparatus 9 do not have to be electrically connected to each other, and thus, noise may be inhibited from being mixed during the air feeding operation of the air feeding apparatus 9. Fig. 3 is a diagram for describing an example configuration of the air feeding apparatus according to the modification of the embodiment, for a case of performing or stopping the air feeding operation according to a measurement value obtained by measuring the internal pressure of the suction pipe.

Furthermore, according to the present modification, the air feeding pipe 30 may be formed at a distal end surface of the distal end portion 23 in such a way that the air feeding opening 302 is provided at a position as shown in Fig. 4. Fig. 4 is a diagram for describing an example configuration of the distal end surface of the distal end portion of the endoscope according to the modification of the embodiment.

More specifically, for example, the air feeding pipe 30 may be formed in such a way that the air feeding opening 302 is provided at a part that belongs to a region AR, of a distal end surface 23A of the distal end portion 23, formed by connecting a center point of a circle corresponding to the objective lens 232 and a circle corresponding to the forceps opening 251 by two tangent lines, and that lies between the objective lens 232 and the forceps opening 251. According to such a configuration of the distal end surface 23A, for example, mist generated by application of electrical energy to a body tissue may be blown off to a position that is separated from the objective lens 232 as much as possible by gas that is discharged from the air feeding opening 302 when the cautery apparatus 8 is in the on state. Accordingly, the configuration of the distal end surface 23A as described above allows the observation field of view of the objective lens 232 to be maintained as clear as possible even during the ESD.

Note that the present invention is not limited to the embodiment described above, and it should be understood that various modifications and applications are possible without departing from the spirit of the invention.

The present application claims priority from Japanese Patent Application No. 2015-094992 filed in Japan on May 7, 2015, the entire contents of which are incorporated in the present specification, claims, and drawings by reference.

## Claims

1. An endoscope system comprising:
an endoscope including an insertion section configured to be inserted into a body cavity of a subject, and an objective lens provided in a distal end portion of the insertion section;
a first pipe provided in the insertion section, the first pipe being configured to cause foreign matters that are a cause of obstruction of an observation field of view of the objective lens to flow outside the endoscope;
a valve provided at an intermediate portion of the first pipe, the valve being configured to switch the first pipe to either one of an open state and a closed state;
a first suction apparatus provided outside the endoscope, the first suction apparatus being configured to perform a suction operation of sucking the foreign matters through the valve in a case where the first pipe is in the open state;
a second pipe formed as a pipe divided from a part, of the intermediate portion of the first pipe, not passing through the valve, the second pipe being configured to cause fluid, in the foreign matters, containing a solid particle component to flow outside the endoscope;
a cautery apparatus configured to perform a surgical treatment on a body tissue in the observation field of view of the objective lens by applying energy to the body tissue; and
a second suction apparatus provided outside the endoscope, the second suction apparatus being configured to perform a suction operation of sucking the fluid through the second pipe when energy is applied to the body tissue by the cautery apparatus.

2. The endoscope system according to claim 1, further comprising:
a third pipe provided in the insertion section, the third pipe being formed as a separate pipe different from the first pipe, and being configured to cause gas supplied from outside the endoscope to flow through and be discharged from the distal end portion of the insertion section; and
an air feeding apparatus provided outside the endoscope, the air feeding apparatus being configured to perform an air feeding operation of supplying gas to the third pipe.

3. The endoscope system according to claim 2, wherein the air feeding apparatus performs the air feeding operation when energy is applied to the body tissue by the cautery apparatus.

4. The endoscope system according to claim 2, wherein the air feeding apparatus performs the air feeding operation when detecting that an internal pressure of the second pipe is less than a predetermined value.

5. The endoscope system according to claim 1, wherein the fluid containing the solid particle component is gas containing a component derived from the body tissue subjected to the surgical treatment by the cautery apparatus.
